# EUROPEAN PATENT APPLICATION

(11) **EP 4 755 901 A1**
(43) Date of publication of application: **10.06.2026**
(21) Application number: 25807891.4
(22) Date of filing: 15.05.2025
(51) Int. Cl.: C07K 14/34, C12N 15/77, C12P 13/06, C07K 14/00

(54) **NOVEL PROMOTER AND METHOD FOR PRODUCING L-ISOLEUCINE BY USING SAME**

(30) Priority: 21.05.2024 KR 20240066104; 15.10.2024 KR 20240140712
(71) Applicant: CJ Cheiljedang Corporation, Seoul 04560 (KR)
(72) Inventor: YOON, Jeong-Hye, Seoul 04560 (KR); KIM, Eunji, Seoul 04560 (KR); JANG, Yongjae, Seoul 04560 (KR); CHOI, Jin-Geun, Seoul 04560 (KR); SHIM, Jihyun, Seoul 04560 (KR); BAE, Jee Yeon, Seoul 04560 (KR)
(74) Representative: Meissner Bolte Partnerschaft mbB
(86) International application number: PCT/KR2025/006594
(87) International publication number: WO 2025/244351

(57) **Abstract**

The present disclosure relates to a novel polynucleotide exhibiting promoter activity, and a method for producing L-isoleucine by using same. A microorganism into which the novel polynucleotide of the present disclosure, in which specific positions of promoter regions of an NCgl2412 gene, an NCgl2046 gene, and/or an NCgl1896 gene are mutated, is introduced has significantly increased L-isoleucine productivity, and thus the novel polynucleotide can be effectively used to efficiently produce L-isoleucine.

## Description

### [TECHNICAL FIELD]

### Cross-reference to related application(s)

The present disclosure claims a benefit of the priority to Korean Patent Application No. 10-2024-0066104 filed on May 21, 2024, and Korean Patent Application No. 10-2024-0140712 filed on October 15, 2024, and the entire contents disclosed in the documents of the corresponding Korean patent applications are incorporated as a part of the present disclosure.

Throughout the present disclosure, numerous papers and patent documents are referenced and citations thereof are indicated. The disclosures of the cited papers and patent documents are incorporated into the present disclosure in their entirety by reference to more clearly describe the level of the technical field to which the present disclosure pertains and the contents of the present invention.

The present disclosure relates to a novel promoter and a method for producing L-isoleucine using the same, and more particularly, to a novel polynucleotide having a promoter activity, an expression cassette comprising the polynucleotide and a target gene operably linked thereto, a vector comprising the polynucleotide or the expression cassette, a microorganism comprising the polynucleotide or the expression cassette, and a method for producing L-isoleucine using the microorganism.

### [BACKGROUND ART]

L-isoleucine (Ile, I) is classified as an essential amino acid, a type of branched-chain amino acid among the total twenty essential amino acids, and is used in the manufacture of various products such as animal feed, food additives, and pharmaceuticals. L-isoleucine has been increasingly used in the fields of intravenous solutions, nutritional supplements, sports nutritional supplements, as well as animal feed, because it functions in energy generation after metabolism, hemoglobin production, blood glucose regulation, generation and repair of muscle, and the like.

Based on this trend, various attempts have been made to improve production ability in methods for producing L-isoleucine using various microorganisms including microorganisms of the genus *Corynebacterium* (US6072083A).

Despite such efforts, the development of technology to improve L-isoleucine production ability is still required.

### [DISCLOSURE]

### [TECHNICAL PROBLEM]

An object of the present disclosure is to provide a novel polynucleotide.

Another object of the present disclosure is to provide an expression cassette comprising the polynucleotide and a target gene.

Another object of the present disclosure is to provide a microorganism comprising the polynucleotide or the polynucleotide and a target gene operably linked thereto.

Another object of the present disclosure is to provide a method for producing L-isoleucine, comprising culturing the microorganism in a medium.

Another object of the present disclosure is to provide a use of the microorganism for producing L-isoleucine.

### [TECHNICAL SOLUTION]

Description thereof in detail is as follows. Meanwhile, each description and embodiment disclosed in the present disclosure may also be applied to other descriptions and embodiments. That is, all combinations of various elements disclosed in the present disclosure fall within the scope of the present disclosure. In addition, it cannot be considered that the scope of the present disclosure is limited by the specific descriptions described below. Furthermore, those skilled in the art will recognize, or be able to ascertain using no more than routine experimentation, many equivalents to the specific aspects of the present disclosure described in the present disclosure. In addition, such equivalents are intended to be included in the present disclosure.

Furthermore, throughout the present disclosure, numerous papers and patent documents are referenced and citations thereof are indicated. The disclosures of the cited papers and patent documents are incorporated into the present disclosure in their entirety by reference to more clearly describe the level of the technical field to which the present disclosure pertains and the contents of the present disclosure.

An aspect of the present disclosure provides a polynucleotide, comprising:
a nucleotide sequence wherein the nucleotide at position 99 is substituted with a nucleotide different from an original nucleotide in the nucleotide sequence of SEQ ID NO: 54;
a nucleotide sequence wherein the nucleotide at position 217 is substituted with a nucleotide different from an original nucleotide, the nucleotide at position 218 is substituted with a nucleotide different from an original nucleotide, the nucleotide at position 219 is substituted with a nucleotide different from an original nucleotide, the nucleotide at position 220 is substituted with a nucleotide different from an original nucleotide, the nucleotide at position 221 is substituted with a nucleotide different from an original nucleotide, the nucleotide at position 224 is substituted with a nucleotide different from an original nucleotide, the nucleotide at position 226 is substituted with a nucleotide different from an original nucleotide, and the nucleotide at position 228 is substituted with a nucleotide different from an original nucleotide in the nucleotide sequence of SEQ ID NO: 58; or
a nucleotide sequence wherein the nucleotide at position 155 is substituted with a nucleotide different from an original nucleotide, the nucleotide at position 163 is substituted with a nucleotide different from an original nucleotide, the nucleotide at position 164 is substituted with a nucleotide different from an original nucleotide, and the nucleotide at position 165 is substituted with a nucleotide different from an original nucleotide in the nucleotide sequence of SEQ ID NO: 60.

In the present disclosure, the nucleotide sequence of SEQ ID NO: 54, SEQ ID NO: 58, or SEQ ID NO: 60 can be retrieved in a known database, NCBI Genbank, and the nucleotide sequence of SEQ ID NO: 54, SEQ ID NO: 58, or SEQ ID NO: 60 may each be derived from *Corynebacterium sp*., and specifically, may be a sequence derived from *Corynebacterium glutamicum.* The nucleotide sequence of SEQ ID NO: 54, SEQ ID NO: 58, or SEQ ID NO: 60 may be an exemplary sequence for specifying a mutation site to be introduced for preparing the polynucleotide of the present disclosure, and it is apparent that the mutation introduced into the polynucleotide of the present disclosure can also be introduced into any sequence that is functionally corresponding to the nucleotide sequence of SEQ ID NO: 54, SEQ ID NO: 58, or SEQ ID NO: 60. In one example, the functionally corresponding sequence may be a nucleotide sequence having at least 70% or more, 80% or more, 85% or more, 90% or more, 95% or more, 96% or more, 97% or more, 98% or more, or 99% or more, and less than 100% homology or identity to the nucleotide sequence of SEQ ID NO: 54, SEQ ID NO: 58, or SEQ ID NO: 60, or a nucleotide sequence having the homology or identity in which some sequences are added, deleted, or modified, but is not limited thereto.

Accordingly, in one example, the polynucleotide of the present disclosure may comprise, consist essentially of, or consist of a nucleotide sequence wherein the nucleotide at position 99 in the nucleotide sequence of SEQ ID NO: 54 is substituted with a nucleotide different from an original nucleotide, and which has at least 70% or more, 80% or more, 85% or more, 90% or more, 95% or more, 96% or more, 97% or more, 98% or more, 99% or more, 99.1% or more, 99.2% or more, 99.3% or more, 99.35% or more, or 99.39% or more; and less than 100% homology or identity to the nucleotide sequence of SEQ ID NO: 54, or a nucleotide sequence having the homology or identity in which some sequences are added, deleted, or modified. In another example, the polynucleotide of the present disclosure may comprise, consist essentially of, or consist of a nucleotide sequence wherein the nucleotides at position 217, position 218, position 219, position 220, position 221, position 224, position 226, and position 228 in the nucleotide sequence of SEQ ID NO: 58 are respectively substituted with a nucleotide different from an original nucleotide, and which has at least 70% or more, 80% or more, 85% or more, 90% or more, 95% or more, 96% or more, 96.1% or more, 96.2% or more, 96.3% or more, 96.4% or more, 96.5% or more, 96.55% or more, or 96.59% or more; and less than 100% homology or identity to the nucleotide sequence of SEQ ID NO: 58, or a nucleotide sequence having the homology or identity in which some sequences are added, deleted, or modified. In another example, the polynucleotide of the present disclosure may comprise, consist essentially of, or consist of a nucleotide sequence wherein the nucleotides at position 155, position 163, position 164, and position 165 in the nucleotide sequence of SEQ ID NO: 60 are respectively substituted with a nucleotide different from an original nucleotide, and which has at least 70% or more, 80% or more, 85% or more, 90% or more, 95% or more, 96% or more, 97% or more, 97.25% or more, 97.5% or more, 97.6% or more, 97.7% or more, 97.8% or more, 97.9% or more, or 98% or more; and less than 100% homology or identity to the nucleotide sequence of SEQ ID NO: 60, or a nucleotide sequence having the homology or identity in which some sequences are added, deleted, or modified.

In the present disclosure, the nucleotide sequence of SEQ ID NO: 54 may be a promoter sequence of the NCgl2412 gene or a part of the promoter sequence.

In one embodiment, the nucleotide at position 99 in the nucleotide sequence of SEQ ID NO: 54 may be guanine, but is not limited thereto.

In one embodiment, the polynucleotide may be a polynucleotide wherein the nucleotide at position 99 in the nucleotide sequence of SEQ ID NO: 54 is substituted with adenine, cytosine, or thymine, and more specifically with cytosine, but is not limited thereto.

In the present disclosure, the nucleotide sequence of SEQ ID NO: 58 may be a promoter sequence of a gene encoding threonine ammonia-lyase IIvA (the NCgl2046 gene, *ilvA*), or a part of the promoter sequence.

The threonine ammonia-lyase IIvA is also called threonine dehydratase (EC 4.3.1.19), is an enzyme producing 2-ketobutyrate from threonine, and is encoded by the *ilvA* gene. A microorganism of the genus *Corynebacterium* synthesizes L-isoleucine via three metabolic intermediates using pyruvate and 2-ketobutyrate as precursors.

In one embodiment, in the nucleotide sequence of SEQ ID NO: 58, the nucleotide at position 217 may be adenine, the nucleotide at position 218 may be adenine, the nucleotide at position 219 may be guanine, the nucleotide at position 220 may be adenine, the nucleotide at position 221 may be thymine, the nucleotide at position 224 may be cytosine, the nucleotide at position 226 may be cytosine, and/or the nucleotide at position 228 may be adenine, but is not limited thereto.

In a specific embodiment, the polynucleotide of the present disclosure may be a polynucleotide wherein the nucleotide at position 217 in the nucleotide sequence of SEQ ID NO: 58 is substituted with cytosine, guanine, or thymine, and more specifically thymine, but is not limited thereto.

In a specific embodiment, the polynucleotide of the present disclosure may be a polynucleotide wherein the nucleotide at position 218 in the nucleotide sequence of SEQ ID NO: 58 is substituted with cytosine, guanine, or thymine, and more specifically guanine, but is not limited thereto.

In a specific embodiment, the polynucleotide of the present disclosure may be a polynucleotide wherein the nucleotide at position 219 in the nucleotide sequence of SEQ ID NO: 58 is substituted with adenine, cytosine, or thymine, and more specifically thymine, but is not limited thereto.

In a specific embodiment, the polynucleotide of the present disclosure may be a polynucleotide wherein the nucleotide at position 220 in the nucleotide sequence of SEQ ID NO: 58 is substituted with cytosine, guanine, or thymine, and more specifically guanine, but is not limited thereto.

In a specific embodiment, the polynucleotide of the present disclosure may be a polynucleotide wherein the nucleotide at position 221 in the nucleotide sequence of SEQ ID NO: 58 is substituted with adenine, cytosine, or guanine, and more specifically, may be a polynucleotide wherein it is substituted with guanine, but is not limited thereto.

In a specific embodiment, the polynucleotide of the present disclosure may be a polynucleotide wherein the nucleotide at position 224 in the nucleotide sequence of SEQ ID NO: 58 is substituted with adenine, guanine, or thymine, and more specifically thymine, but is not limited thereto.

In a specific embodiment, the polynucleotide of the present disclosure may be a polynucleotide wherein the nucleotide at position 226 in the nucleotide sequence of SEQ ID NO: 58 is substituted with adenine, guanine, or thymine, and more specifically adenine, but is not limited thereto.

In a specific embodiment, the polynucleotide of the present disclosure may be a polynucleotide wherein the nucleotide at position 228 in the nucleotide sequence of SEQ ID NO: 58 is substituted with cytosine, guanine, or thymine, and more specifically guanine, but is not limited thereto.

In the present disclosure, the nucleotide sequence of SEQ ID NO: 60 may be a promoter sequence of a gene encoding 4-hydroxy-tetrahydrodipicolinate synthase (EC 4.3.3.7) (NCgl1896 gene, *dapA*) or a part of the promoter sequence.

The 4-hydroxy-tetrahydrodipicolinate synthase is an enzyme involved in producing 4-hydroxy-2,3,4,5-tetrahydrodipicolinate (HTPA) by catalyzing a condensation reaction of pyruvate and aspartate-semialdehyde, and plays an important role in lysine biosynthesis.

In an embodiment, in the nucleotide sequence of SEQ ID NO: 60, the nucleotide at position 155 may be guanine, the nucleotide at position 163 may be thymine, the nucleotide at position 164 may be adenine, and/or the nucleotide at position 165 may be adenine, but is not limited thereto.

In a specific embodiment, the polynucleotide of the present disclosure may be a polynucleotide wherein the nucleotide at position 155 in the nucleotide sequence of SEQ ID NO: 60 is substituted with adenine, cytosine, or thymine, and more specifically thymine, but is not limited thereto.

In a specific embodiment, the polynucleotide of the present disclosure may be a polynucleotide wherein the nucleotide at position 163 in the nucleotide sequence of SEQ ID NO: 60 is substituted with adenine, cytosine, or guanine, and more specifically guanine, but is not limited thereto.

In a specific embodiment, the polynucleotide of the present disclosure may be a polynucleotide wherein the nucleotide at position 164 in the nucleotide sequence of SEQ ID NO: 60 is substituted with cytosine, guanine, or thymine, and more specifically guanine, but is not limited thereto.

In a specific embodiment, the polynucleotide of the present disclosure may be a polynucleotide wherein the nucleotide at position 165 in the nucleotide sequence of SEQ ID NO: 60 is substituted with cytosine, guanine, or thymine, and more specifically guanine, but is not limited thereto.

In one embodiment, the polynucleotide may comprise, consist essentially of, or consist of a nucleotide sequence having at least 70% or more, 80% or more, 85% or more, 90% or more, 95% or more, 96% or more, 97% or more, 98% or more, 99% or more, 99.1 % or more, 99.2% or more, 99.3% or more, 99.35% or more, or 99.39% or more; and less than 100% homology or identity to the nucleotide sequence of SEQ ID NO: 54. More specifically, the polynucleotide may be a polynucleotide wherein the nucleotide at position 99 of the nucleotide sequence of SEQ ID NO: 54 is substituted with cytosine (C), and which comprises, consists essentially of, or consists of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, 99.35% or more, or 99.39% or more; and less than 100% homology or identity to the nucleotide sequence of SEQ ID NO: 54.

In one embodiment, the polynucleotide may comprise, consist essentially of, or consist of a nucleotide sequence having at least 70% or more, 80% or more, 85% or more, 90% or more, 95% or more, 96% or more, 96.1 % or more, 96.2% or more, 96.3% or more, 96.4% or more, 96.5% or more, 96.55% or more, or 96.59% or more; and less than 100% homology or identity to the nucleotide sequence of SEQ ID NO: 58. More specifically, the polynucleotide may comprise, consist essentially of, or consist of a nucleotide sequence wherein, in the nucleotide sequence of SEQ ID NO: 58, the nucleotide at position 217 is substituted with thymine (T), the nucleotide at position 218 is substituted with guanine (G), the nucleotide at position 219 is substituted with thymine (T), the nucleotide at position 220 is substituted with guanine (G), the nucleotide at position 221 is substituted with guanine (G), the nucleotide at position 224 is substituted with thymine (T), the nucleotide at position 226 is substituted with adenine (A), and the nucleotide at position 228 is substituted with guanine (G), and which has at least 70% or more, 80% or more, 85% or more, 90% or more, 95% or more, 96% or more, 96.1% or more, 96.2% or more, 96.3% or more, 96.4% or more, 96.5% or more, 96.55% or more, or 96.59% or more; and less than 100% homology or identity to the nucleotide sequence of SEQ ID NO: 58.

In one embodiment, the polynucleotide may comprise, consist essentially of, or consist of a nucleotide sequence having at least 70% or more, 80% or more, 85% or more, 90% or more, 95% or more, 96% or more, 97% or more, 97.25% or more, 97.5% or more, 97.6% or more, 97.7% or more, 97.8% or more, 97.9% or more, or 98% or more; and less than 100% homology or identity to the nucleotide sequence of SEQ ID NO: 60. More specifically, the polynucleotide may comprise, consist essentially of, or consist of a nucleotide sequence wherein, in the nucleotide sequence of SEQ ID NO: 60, the nucleotide at position 155 is substituted with thymine, the nucleotide at position 163 is substituted with guanine, the nucleotide at position 164 is substituted with guanine, and the nucleotide at position 165 is substituted with guanine, and which has at least 70% or more, 80% or more, 85% or more, 90% or more, 95% or more, 96% or more, 97% or more, 97.25% or more, 97.5% or more, 97.6% or more, 97.7% or more, 97.8% or more, 97.9% or more, or 98% or more; and less than 100% homology or identity to the nucleotide sequence of SEQ ID NO: 60.

In the present disclosure, the polynucleotide may comprise the nucleotide sequence of SEQ ID NO: 3, SEQ ID NO: 7, or SEQ ID NO: 9. In another embodiment, the polynucleotide of the present disclosure may essentially consist of the nucleotide sequence of SEQ ID NO: 3, SEQ ID NO: 7, or SEQ ID NO: 9. In yet another embodiment, the polynucleotide of the present disclosure may consist of the nucleotide sequence of SEQ ID NO: 3, SEQ ID NO: 7, or SEQ ID NO: 9.

In the present disclosure, the term "polynucleotide" may refer to one in which 2 or more, 5 or more, 10 or more, 13 or more, 20 or more, or 30 or more nucleotide monomers are included and the nucleotide monomers are covalently linked to form a chain form.

The polynucleotide of the present disclosure may have a promoter activity and may be used as a universal promoter.

In one embodiment, the polynucleotide may have a promoter activity for the expression of a target gene in microorganisms of the genus *Corynebacterium.*

The polynucleotide having promoter activity may be used interchangeably with "variant promoter" in the present disclosure.

The polynucleotide according to one embodiment of the present disclosure may be utilized as a synthetic promoter having strong activity of inducing expression.

In the present disclosure, the term "promoter" may refer to a DNA region that includes a binding site for a polymerase and initiates transcription of a target gene located at its downstream. The promoter may be located upstream of a transcription start site. The promoter may be operably and/or regulably (enhancing or weakening expression) linked upstream of the target gene. For example, the promoter may be linked in a forward direction on the upstream side of the target gene to enhance (increase) the expression of the gene, or may be linked in a reverse direction on the downstream side of the target gene to weaken (decrease) the expression of the gene. When a promoter is introduced in a reverse direction to downstream of a target gene, for example, downstream of a stop codon, specifically between the stop codon and the top of a transcription terminator, it may weaken the expression of the gene by inducing transcription in the direction opposite to the normal transcription direction of the corresponding gene, thereby causing an RNA polymerase complex to collide with an RNA polymerase complex of the normal direction during the transcription process.

The polymerase, also called RNA polymerase or DNA-dependent RNA polymerase, may refer to an enzyme that synthesizes a primary transcript RNA from DNA. The polymerase may be an RNA polymerase of a prokaryotic cell or an RNA polymerase of a eukaryotic cell (for example, RNA polymerase I, RNA polymerase II, RNA polymerase III, RNA polymerase IV, or RNA polymerase V, etc.). The polynucleotide according to one embodiment may be natural or non-natural, and for example, may be non-natural one which is synthesized chemically or recombinantly.

In the present disclosure, the term "variation" refers to a genetically or non-genetically stable phenotypic change, and may be used interchangeably with "mutation" in the present disclosure.

The polynucleotide (variant promoter) of the present disclosure may have changed (increased or decreased) promoter activity compared to a polynucleotide that does not include a variation (a wild-type polynucleotide or a polynucleotide prior to mutation). The polynucleotide may regulate (increase or decrease) the expression of a target gene operably linked thereto, or the expression or activity of a protein encoded by the target gene, and furthermore, may regulate the expression of other genes besides the target gene.

The "target gene" means a gene whose expression is intended to be regulated by the polynucleotide of the present disclosure, and in the case of a gene encoding a protein, may be used interchangeably with 'a gene encoding a target protein'. A protein encoded by the target gene may be expressed as a "target protein", and a gene encoding the "target protein" may be expressed as a "target gene".

The amino acid coding sequence of the target gene may be variously modified within a range that does not change the protein sequence encoded by the target gene, due to the degeneracy of the codon or in consideration of a preferred codon (codon usage frequency) in an organism in which the target gene is to be expressed.

The polynucleotide of the present disclosure, when introduced into a suitable host cell together with a target gene operably linked thereto, may have an activity of increasing the production ability (production amount) of a target substance of the host cell, for example, the production ability (production amount) of an amino acid.

In one embodiment, the polynucleotide may be for increasing amino acid production ability (production amount), and specifically, may be for increasing the production ability (production amount) of L-isoleucine.

In addition, the nucleotide sequence of the polynucleotide may be further modified by conventionally known mutagenesis methods, for example, directed evolution and site-directed mutagenesis, and the like, to the extent that it maintains a corresponding biological activity (promoter activity) and/or a desired activity (e.g., an activity of increasing production of a target substance in a host cell).

Therefore, the polynucleotide of the present disclosure may be a polynucleotide that comprises, consists essentially of, or consists of a nucleotide sequence having at least 70%, 80%, 85%, 90%, 95%, 96%, 97%, 98%, 99%, 99.5%, 99.7% or 99.9% or more homology or identity to the nucleotide sequence of SEQ ID NO: 3, the nucleotide sequence of SEQ ID NO: 7, or the nucleotide sequence of SEQ ID NO: 9, or a nucleotide sequence having the homology or identity in which some sequences are added, deleted, or modified.

In the present disclosure, the term, 'homology' or 'identity' means the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and default gap penalties established by the program used may be employed together. Substantially, homologous or identical sequences can generally hybridize with the entire sequence or a portion thereof under medium or high stringent conditions. It is apparent that hybridization also includes hybridization with a polynucleotide containing a general codon or codon by considering the codon degeneracy in the polynucleotide.

Whether any two polynucleotide or polypeptide sequences have homology or identity may be determined, for example, by using a known computer algorithm such as the "FASTA" program using default parameters as in Pearson et al (1988) [Proc.Natl. Acad. Sci. USA 85]: 2444. Alternatively, the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or a later version), may be used for the determination (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073)). For example, homology or identity may be determined using BLAST of the National Center for Biotechnology Information (NCBI) database, or ClustalW.

The homology or identity of a polynucleotide or a polypeptide may be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program can be defined as the value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for end gaps.

In one example, a polynucleotide comprising a specific nucleotide sequence provided in the present disclosure may be interpreted as comprising not only the specific nucleotide sequence or a nucleotide sequence substantially equivalent thereto, but also a polynucleotide fragment comprising a nucleotide sequence complementary to the specific nucleotide sequence. Specifically, the polynucleotide having the complementarity can be identified under the conditions described below: these conditions are specifically described in known literature. For example, conditions under which genes having high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more, hybridize with each other, and the genes having lower complementarity do not hybridize, or conditions of washing once, specifically two to three times, at a salt concentration and temperature corresponding to the washing conditions of a conventional Southern hybridization, i.e., 60°C, 1x SSC (saline-sodium citrate buffer), and 0.1% (w/v) SDS (Sodium Dodecyl Sulfate); 60°C, 0.1x SSC, and 0.1% SDS; or 68°C, 0.1x SSC, and 0.1% SDS, may be listed, but are not limited thereto. Hybridization requires that two nucleotides have complementary sequences, but some base mismatches may be allowed depending on the stringency of hybridization. The term "complementary" may be used to describe the relationship between nucleotide that can hybridize with each other. For example, in the case of DNA, adenosine is complementary to thymine and cytosine is complementary to guanine. The stringency of hybridization between polynucleotides depends on the length and degree of complementarity of the polynucleotides, which is well known in the relevant art (see Sambrook et al., supra, 9.50-9.51, 11.7-11.8).

Furthermore, the polynucleotide of the present disclosure may be operably linked to a gene encoding a target protein, i.e., a target gene.

In the present disclosure, the term "operably linked (operatively linked)" means that a polynucleotide having promoter activity of the present disclosure is functionally linked to the gene sequence so as to initiate and mediate transcription of the target gene. An operative linkage can be prepared using known gene recombination techniques in the art, and site-specific DNA cleavage and ligation can be produced using cleavage and ligation enzymes and the like in the art, but are not limited thereto.

When the polynucleotide is operably linked to the target gene, some nucleotides may be added, deleted, and/or modified for the use of the cleavage and ligation enzymes and the like.

In one embodiment, the target gene may be a gene encoding a protein involved in the production of L-isoleucine of the present disclosure, but is not limited thereto. The protein involved in the production of L-isoleucine may be a protein involved in at least one process or step of an intracellular production pathway (e.g., biosynthesis, metabolism, bioconversion, etc.), intracellular transport, and/or an extracellular excretion pathway of L-isoleucine, and may be selected from the group consisting of, for example, an enzyme (various synthases, lyases, kinases, carboxylases (e.g., pyruvate carboxylase, etc.), reductases, oxidases, decarboxylases, dehydrogenases, dehydratases, transferases, epimerases, etc.), an intermediate, a transport protein, a membrane protein (channel, etc.), and the like, but is not limited thereto.

In one embodiment, the target gene may be the NCgl2412 gene, but is not limited thereto.

In one embodiment, the target gene may be the NCgl2046 gene, but is not limited thereto. The NCgl2046 gene may be a gene encoding threonine ammonia-lyase IIvA (*ilvA*).

In one embodiment, the target gene may be the NCgl1896 gene, but is not limited thereto. The NCgl1896 gene may be a gene encoding 4-hydroxy-tetrahydrodipicolinate synthase (*dapA*).

Another aspect of the present disclosure provides an expression cassette comprising a polynucleotide of the present disclosure and a target gene.

The polynucleotide and target gene are as described above.

In the present disclosure, the term, "expression cassette," refers to a unit cassette that comprises a promoter and a target gene operably linked thereto, and is capable of expressing a target gene located downstream of the promoter. Inside or outside such a gene expression cassette, various factors that can aid in the efficient expression of the target gene may be included. The gene expression cassette, in addition to the promoter operably linked to the target gene, may typically include a transcription termination signal, a ribosome binding site, and a translation termination signal, but is not limited thereto.

Another aspect of the present disclosure provides a vector comprising the polynucleotide; the polynucleotide and a target gene operably linked thereto; or the expression cassette of the present disclosure.

The polynucleotide, the target gene, and the expression cassette are as described above. The vector may include a target gene operably linked to the polynucleotide.

In the present disclosure, the term "vector" refers to a DNA construct that contains the nucleotide sequence of the polynucleotide encoding the target protein, operably linked to a suitable regulatory sequence, so as to be able to express the target protein in a suitable host. The regulatory sequence may include a promoter capable of initiating transcription, any operator sequence for regulating transcription, a sequence encoding a suitable mRNA ribosome binding site, and/or a sequence that regulates the termination of transcription and/or translation. After being transformed into a suitable host cell, a vector may be expressed independently of the genome of the host cell or may be integrated into the genome of the host cell.

A vector usable in the present disclosure is not particularly limited as long as it is replicable in a host cell, and may be selected from among all commonly used vectors. Examples of commonly used vectors include plasmids, cosmids, viruses, bacteriophages, and the like, in a natural or recombinant state. For example, as the vector, pWE15, M13, MBL3, MBL4, IXII, ASHII, APII, t10, t11, Charon4A, and Charon21A, and the like may be used as phage vectors or cosmid vectors, and pDZ series, pBR series, pUC series, pBluescriptll series, pGEM series, pTZ series, pCL series, and pET series, and the like may be used as plasmid vectors. Specifically, examples may include, but are not limited to, pDZ, pDC, pACYC177, pACYC184, pCL, pECCG117, pUC19, pBR322, pMW118, pCC1BAC, pDCM2, pDC24 vectors, and the like.

A vector usable in the present disclosure may be an expression vector or an insertion vector for integration into a host cell chromosome. The insertion of the target DNA into the host cell chromosome using an insertion vector may be performed by any method known in the art, for example, by homologous recombination or a CRISPR system, but is not limited thereto. The vector may further include a selection marker for confirming whether the vector has been transformed or further whether the target DNA has been inserted into the chromosome. The selection marker may be selected and used from among genes that confer a selectable phenotype, such as drug resistance, auxotrophy, resistance to cytotoxic agents, or expression of surface proteins. In an environment treated with a selective agent, only cells expressing the selection marker can survive or exhibit a different phenotype, and thus, transformed cells can be selected.

In the present disclosure, the term "transformation" refers to introducing a target polynucleotide into a host cell. A transformed polynucleotide may be inserted into the chromosome of the host cell or may be located extrachromosomally. Furthermore, the polynucleotide may be DNA and/or RNA, and regardless of the form in which it is introduced, as long as it can be introduced into a host cell and function therein. For example, the polynucleotide may be introduced into a host cell in the form of an expression cassette, which is a gene construct including all elements necessary for its own expression, or in the form of a vector including the same.

The transformation method includes any method for introducing the target polynucleotide into a cell, and may be performed by selecting a suitable standard technique as is known in the art, depending on the host cell. For example, examples include electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method, and the like, but it is not limited thereto.

Another aspect of the present disclosure provides a microorganism (host cell) comprising the polynucleotide, the polynucleotide and a target gene operably linked thereto, or the expression cassette of the present disclosure.

The polynucleotide, the target gene, or the expression cassette are as described above.

The polynucleotide, the polynucleotide and the target gene operably linked thereto, or the expression cassette may be introduced into a microorganism by transformation, but is not limited thereto.

In the present disclosure, the term "microorganism" includes both a wild-type microorganism and a microorganism that has been naturally or artificially genetically modified, and also includes a microorganism in which a specific mechanism is attenuated or enhanced due to causes such as the insertion of an exogenous gene or the enhancement or attenuation of the activity of an endogenous gene.

The microorganism may be a microorganism that naturally expresses a target gene or a microorganism that has a production ability of target product, or may be a microorganism in which an expression ability of target gene or a production ability of target product is conferred to a parent strain that does not naturally express a target gene or does not have a production ability of target product, but is not limited thereto. In one embodiment, the microorganism may be a microorganism that naturally produces an amino acid, specifically a microorganism that produces L-isoleucine.

In the present disclosure, the term "target product" refers to a biologically active substance that is intended to be produced or whose production is intended to be regulated (increased or decreased) by using the polynucleotide, the polynucleotide and a target gene operably linked thereto, an expression cassette comprising the polynucleotide and the target gene, a vector comprising the same, and/or a microorganism comprising the same provided in the present disclosure, and is intended to include not only the biologically active substance to be finally produced but also a target protein that can be produced by the microorganism. For example, it may refer to the target protein itself encoded by the target gene, and/or any biologically active substance produced through the involvement of the target protein. The biologically active substance refers to any substance that is produced or derived from an organism (e.g., a cell) or has a predetermined function in vivo or within a cell, and may be, for example, an amino acid (glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, O-acetyl homoserine, etc.), a nucleic acid, a vitamin (vitamin A, B (B1, B2, B3, B5, B6, B7, B9, B12, etc.), C, D, E, K, etc.), a protein (the target protein or a protein other than it, for example, a hormone, a growth factor, a cytokine, an immunoglobulin (antibody), an antigen protein, a receptor, a ligand, a functional fragment thereof (a fragment retaining a desired function), a fusion protein in which two or more are fused, etc.), a sugar (e.g., a monosaccharide, a disaccharide, a polysaccharide, a sugar alcohol, etc.), a fatty acid (myristoleic acid, palmitoleic acid, sapienic acid, oleic acid, elaidic acid, vaccenic acid, linoleic acid, linoelaidic acid, arachidonic acid, eicosapentaenoic acid (EPA), erucic acid, docosahexaenoic acid (DHA), etc.), an organic acid (lactic acid, citric acid, oxalic acid, uric acid, butyric acid, stearic acid, propionic acid, etc.), and the like, but is not limited thereto. In addition, if it is a substance produced through the involvement of the target protein, metabolites thereof (polyhydroxyalkanoate (PHA), etc.), precursors thereof, derivatives thereof that maintain their biological activity, and the like may also be included in the target product, in addition to the substances described above.

In one embodiment, the microorganism comprising the polynucleotide and a target gene operably linked thereto of the present disclosure may be a microorganism with an increased production ability of an amino acid as a target product, and specifically may be a microorganism with an increased production ability of L-isoleucine. The microorganism with the increased production ability of L-isoleucine may be a microorganism with an increased production ability of L-isoleucine compared to a microorganism that does not comprise the polynucleotide of the present disclosure, for example, a microorganism in which the expression of the NCgl2412 gene, the NCgl2046 gene, or the NCgl1896 gene is regulated by a polynucleotide prior to introduction of a mutation, but is not limited thereto. The polynucleotide before the introduction of the mutation may be a wild-type polynucleotide, and specifically may consist of the nucleotide sequence of SEQ ID NO: 54, SEQ ID NO: 58, or SEQ ID NO: 60.

In the present disclosure, a "unmodified microorganism" does not exclude a strain including a naturally occurring mutation, and may refer to a wild-type strain or a naturally occurring strain itself, or a strain before its properties are changed by genetic variation due to natural or artificial factors. For example, the unmodified microorganism may refer to a strain in which a variant polynucleotide has not been introduced into the promoter region of the NCgl2412 gene, the NCgl2046 gene, or the NCgl1896 gene described in the present disclosure, or a strain before such introduction. The "unmodified microorganism" may be used interchangeably with "pre-modification strain", "pre-modification microorganism", "non-mutant strain", "unmodified strain", "non-mutant microorganism", or "reference microorganism".

In one embodiment, the microorganism as a host cell may be *Corynebacterium glutamicum* KCCM12739P (CA10-3101, US 2023-0098971 A1) or *Corynebacterium glutamicum* KCCM11248P (Korean Patent No. 10-1335789), but is not limited thereto.

In one embodiment, the microorganism (a variant microorganism or a microorganism as a host cell) may be a microorganism in which the biosynthetic pathway of L-isoleucine is additionally enhanced to increase the production amount of L-isoleucine, but is not limited thereto.

In one example, the microorganism may be a microorganism in which feedback inhibition of L-threonine dehydratase is released, and/or feedback inhibition of homoserine dehydrogenase is released.

In one embodiment, the microorganism may be a microorganism in which feedback inhibition of L-threonine dehydratase is released.

In the present disclosure, the term "threonine dehydratase (EC 4.3.1.19)" is an enzyme that produces 2-ketobutyrate from threonine, is encoded by the ilvA gene, and is known to be subjected to feedback inhibition by L-isoleucine. A microorganism of the genus *Corynebacterium* synthesizes L-isoleucine via three intermediate metabolites using pyruvate and 2-ketobutyrate as precursors. The amino acid sequence of the threonine dehydratase can be obtained from GenBank of NCBI, which is a known database, or from US 2023-0098971 A1, US 10982244 B2.

In another embodiment, the microorganism may be a microorganism in which feedback inhibition of homoserine dehydrogenase is released.

In the present disclosure, the term "homoserine dehydrogenase (EC:1.1.1.3)" is an enzyme which is encoded by the *hom* gene and catalyzes the synthesis of homoserine. Homoserine dehydrogenase is known to be subjected to feedback inhibition by isoleucine. The amino acid sequence of the homoserine dehydrogenase can be obtained from GenBank of NCBI, which is a known database, or from US 10982244 B2.

In one embodiment, the microorganism may be a microorganism in which feedback inhibition of aspartate kinase is released. In the present disclosure, the term "aspartate kinase (EC: 2.7.2.4)" is encoded by the lysC gene, and the amino acid sequence of the aspartate kinase can be obtained from GenBank of NCBI, which is a known database, or from US 10662450 B2.

In the present disclosure, the term, release of feedback inhibition, may mean that the activity of a polypeptide, protein, or enzyme is increased or enhanced compared to its intrinsic activity, or is no longer subject to inhibition relative to its intrinsic activity. In one embodiment, the microorganism may be a microorganism that further includes one or more mutations among the mutations of: additionally introducing a genetic mutation (R407H) into the *hom* gene (US 10982244 B2); additionally introducing a genetic mutation (T381A, F383A and/or V323A) into the *ilvA* gene (US 2023-0098971 A1, US 10982244 B2); and additionally introducing a genetic mutation (L377K) into the *lysC* gene encoding aspartate kinase (US 10662450 B2), but is not limited thereto.

The microorganism of the present disclosure can include, without limitation, any microorganism into which the polynucleotide of the present disclosure can be introduced and function as a promoter.

In one embodiment, the microorganism may be a microorganism of the genus *Corynebacterium* (*Corynebacterium* sp.), a microorganism of the genus *Escherichia*(*Escherichia* sp.), or a microorganism of the genus *Bacillus* (*Bacillus* sp.), but is not limited thereto.

Specifically, the microorganism may be a microorganism of the genus *Corynebacterium,* and more specifically, may include *Corynebacterium glutamicum, Corynebacterium stationis, Corynebacterium thermoaminogenes, Corynebacterium glutamicum, Brevibacterium flavum, Brevibacterium lactofermentum,* and strains prepared therefrom, but is not limited thereto. Specifically, the microorganism of the genus *Corynebacterium* may be *Corynebacterium glutamicum.*

In one embodiment, the microorganism may be *Corynebacterium glutamicum* in which a mutation has been introduced into the promoter of the NCgl2412 gene, the NCgl2046 gene, or the NCgl1896 gene, but is not limited thereto.

In one embodiment, the microorganism of the present disclosure with increased L-isoleucine production ability (production amount) may have an L-isoleucine production ability (production amount) increased by about 5% or more, about 6% or more, about 8% or more, about 9% or more, about 10% or more, about 20% or more, about 30% or more, about 40% or more, about 45% or more, about 50% or more, about 60% or more, about 70% or more, about 75% or more, or about 80% or more (the upper limit is not particularly limited, and may be, for example, about 200% or less, about 150% or less, about 100% or less, or about 90% or less) compared to a parent strain before mutation or an unmodified microorganism, but is not limited thereto. In another embodiment, the microorganism of the present disclosure with increased L-isoleucine production ability (production amount) may have an L-isoleucine production ability (production amount) increased by about 1.05-fold or more, about 1.06-fold or more, about 1.08-fold or more, about 1.09-fold or more, about 1.1-fold or more, about 1.2-fold or more, about 1.3-fold or more, about 1.4-fold or more, about 1.45-fold or more, about 1.5-fold or more, about 1.6-fold or more, about 1.7-fold or more, about 1.75-fold or more, or about 1.8-fold or more (the upper limit is not particularly limited, and may be, for example, about 10-fold or less, about 5-fold or less, about 3-fold or less, about 2-fold or less, or about 1.5-fold or less) compared to a parent strain before mutation or an unmodified microorganism, but is not limited thereto.

The term "about" is a range including all of ±0.5, ±0.4, ±0.3, ±0.2, ±0.1, etc., and includes all numerical values in a range equivalent or similar to the numerical value that follows the term about, but is not limited thereto.

Another aspect of the present disclosure provides a composition for producing L-isoleucine, comprising at least one selected from the group consisting of the polynucleotide, the polynucleotide and a target gene operably linked thereto, the expression cassette, the vector, and the microorganism of the present disclosure. The polynucleotide, the target gene, the expression cassette, the vector, the microorganism, and the like are as described above.

In one example, the composition for producing L-isoleucine may further comprise any suitable excipient conventionally used in a composition for producing L-isoleucine, and such an excipient may be, for example, a preservative, a wetting agent, a dispersing agent, a suspending agent, a buffer, a stabilizer, or an isotonic agent, and the like, but is not limited thereto.

Another aspect of the present disclosure provides a use, of at least one selected from the group consisting of the polynucleotide, the polynucleotide and a target gene operably linked thereto, the expression cassette, the vector, and the microorganism of the present disclosure for producing L-isoleucine. The polynucleotide, the target gene, the expression cassette, the vector, the microorganism, and the like are as described above.

Another aspect of the present disclosure provides a use of at least one selected from the group consisting of the polynucleotide, the polynucleotide and a target gene operably linked thereto, the expression cassette, the vector, and the microorganism of the present disclosure for preparing a composition for producing L-isoleucine. The polynucleotide, the target gene, the expression cassette, the vector, the microorganism, and the like are as described above.

Another aspect of the present disclosure provides a method for producing a target product, the method comprising a step of culturing in a medium a microorganism comprising the polynucleotide, the polynucleotide and a target gene operably linked thereto, the expression cassette or the vector of the present disclosure.

The polynucleotide, the target gene, the expression cassette, the vector, the microorganism, and the target product are as described above.

In one embodiment, the target product may be an L-amino acid. Specifically, the target product may be L-isoleucine.

The method may further comprise a step of recovering the target product from the cultured microorganism, the culture, or both, after the step of culturing.

The term "culturing" in the present disclosure means growing a microorganism under appropriately and artificially controlled environmental conditions. The culturing process of the present disclosure may be performed according to a suitable medium and culture conditions known in the art. Such a culturing process can be easily adjusted and used by a person skilled in the art according to a selected strain. Specifically, the culturing may be a batch, continuous, and/or fed-batch culture, but is not limited thereto. These various methods are disclosed in, for example, "Biochemical Engineering" (James M. Lee, Prentice-Hall International Editions, pp138-176, 1991).

In the present disclosure, the term "medium" means a substance prepared by mixing nutrients required for culturing the microorganism of the present disclosure as main components, and supplies water, which is essential for survival and growth, as well as nutrients and growth factors. Specifically, as the medium and other culture conditions used for culturing the microorganism of the present disclosure, any medium used for culturing conventional microorganisms can be used without particular limitation, but the microorganism of the present disclosure can be cultured in a conventional medium containing a suitable carbon source, nitrogen source, phosphorus source, inorganic compound, amino acid and/or vitamin, while controlling temperature, pH, etc., under aerobic conditions. Specifically, a culture medium for a microorganism can be found in literature such as ["Manual of Methods for General Bacteriology" by the American Society for Bacteriology (Washington D.C., USA, 1981)].

The medium used for culturing must meet the requirements of a specific strain in an appropriate manner. For example, culturing may be performed in a conventional medium containing a suitable carbon source, nitrogen source, amino acid, vitamin, etc., while controlling temperature, pH, etc., under aerobic conditions. At this time, the carbon source includes carbohydrates such as glucose, fructose, and sucrose, and amino acids such as glutamic acid and cysteine. Specifically, natural organic nutrient sources such as starch hydrolysates and molasses can be used, and preferably carbohydrates such as glucose, fructose, sterilized pre-treated molasses (i.e., molasses converted to reducing sugars), and other appropriate amounts of carbon sources can be used in various ways without limitation, but are not limited thereto. As the nitrogen source, inorganic nitrogen sources such as ammonia; and amino acids such as glutamic acid and cysteine, and peptone, meat extract, yeast extract, etc., can be used as organic nitrogen sources. These nitrogen sources may be used alone or in combination, but are not limited thereto. In the medium, phosphoric acid, potassium dihydrogen phosphate or dipotassium hydrogen phosphate, or a corresponding sodium-containing salt may be used as a phosphorus source, but is not limited thereto. As the inorganic compound, magnesium sulfate, iron sulfate, manganese sulfate, and calcium chloride, etc., may be used, and in addition, amino acids, vitamins, and appropriate precursors, etc., may be included. These media or precursors may be added to the culture in a batch or continuous manner, but are not limited thereto.

During culturing, the pH of the culture can be adjusted by adding compounds such as potassium hydroxide, ammonia, and phosphoric acid to the culture in an appropriate manner. Furthermore, during culturing, an antifoaming agent such as a fatty acid polyglycol ester can be used to suppress foam generation. Furthermore, to maintain the aerobic state of the culture, oxygen or an oxygen-containing gas can be injected into the culture. The temperature of the culture is 27°C to 37°C, and specifically 30°C to 33°C. The culturing period may be continued until the desired production amount of the useful substance is obtained, and is specifically 10 to 160 hours.

In the present disclosure, the term "culture" means a substance comprising a medium in which a microorganism is growing or has completed growth under appropriately and artificially controlled environmental conditions. In a narrow sense, the culture does not include the grown microorganism, but it may be included in a broad sense. The "culture" may include various target substances discharged by the microorganism into the medium during its growth, along with the medium components formulated for the microorganism culture.

In the culturing of the present disclosure, the culture temperature may be maintained at 20°C to 45°C, specifically 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, and the culturing may be performed for about 10 to 160 hours, 20 to 100 hours, 30 to 90 hours, or 50 to 70 hours, but is not limited thereto.

The target product produced by the culturing of the present disclosure may be secreted into the medium or may be retained within the cells.

The step of recovering the target product may be to collect the target product using a suitable method known in the art, according to the culturing method, for example, a batch, continuous, or fed-batch culturing method. For example, various types of chromatography such as centrifugation, filtration, treatment with a crystallizing protein precipitating agent (salting out), extraction, ultrasonic disruption, ultrafiltration, dialysis, molecular sieve chromatography (gel filtration), adsorption chromatography, ionexchange chromatography, and affinity chromatography, HPLC, or a combination of these methods may be used, but is not limited thereto, and the target product can be recovered from the medium or the microorganism using a suitable method known in the art.

Furthermore, the method for producing a target product of the present disclosure may additionally comprise a purification step. The purification can be performed using a suitable method known in the art. In one example, when the method for producing a target product of the present disclosure comprises both a recovery step and a purification step, the recovery step and the purification step may be performed separately (or continuously) regardless of the order, or may be performed simultaneously or integrated into a single step, but is not limited thereto.

Another aspect of the present disclosure provides a method for increasing the production ability of a target product, the method comprising a step of culturing in a medium a microorganism comprising the polynucleotide, the polynucleotide and a target gene, or an expression cassette comprising the polynucleotide and a target gene of the present disclosure.

The polynucleotide, the target gene, the expression cassette, the microorganism, and the target product are as described above.

Another aspect of the present disclosure provides a method for preparing a microorganism with increased production ability of a target product, the method comprising a step of introducing into a microorganism, the polynucleotide, the polynucleotide and a target gene, or an expression cassette comprising the polynucleotide and a target gene of the present disclosure.

The polynucleotide, the target gene, the expression cassette, the microorganism, and the target product are as described above.

According to another aspect of the present disclosure, the present disclosure provides a composition, a method, a product, a process, or a use characterized by one or more elements disclosed in the present disclosure.

### [ADVANTAGEOUS EFFECTS]

The present disclosure relates to a novel polynucleotide having a promoter activity and a method for producing L-isoleucine using the same, and as the L-isoleucine production ability is significantly increased in a microorganism introduced with the novel polynucleotide of the present disclosure, the novel polynucleotide can be usefully utilized for efficiently producing L-isoleucine.

### [MODE FOR INVENTION]

Hereinafter, the present application will be described in more detail through examples. These examples are intended solely to illustrate the present application more specifically, and it will be apparent to those skilled in the art that the scope of the present application is not limited by these examples, in accordance with the gist of the present application.

### EXAMPLE

(Throughout this specification, the term "%" used to indicate the concentration of a specific substance, unless otherwise specified, refers to (weight/weight) % for solid/solid, (weight/volume) % for solid/liquid, and (volume/volume) % for liquid/liquid.)

The present invention is further described below with reference to the following examples. However, these examples are intended only to illustrate the present invention, and the scope of the present invention is not limited by these examples.

### EXAMPLE 1: Selection of mutant strain with increased isoleucine production ability by artificial mutagenesis

### EXAMPLE 1-1: Random mutagenesis via UV irradiation

In order to select a mutant strain with increased isoleucine production ability, the isoleucine-producing strain of *Corynebacterium glutamicum* KCCM12739P was spread on a nutrient medium containing agar and cultured at 30°C for 16 hours. The hundreds of colonies obtained were irradiated with UV (Ultraviolet mutation) at room temperature to induce random mutations in the genome of the strain.

### <Nutrient Medium (pH 7.2)>

Glucose 10g, meat extract 5g, polypeptone 10g, sodium chloride 2.5g, yeast extract 5g, agar 20g, urea 2g (based on 1 L of distilled water)

### EXAMPLE 1-2: Selection of strains with increased L-isoleucine production ability

In order to select a mutant strain with increased isoleucine production ability compared to the parent strain KCCM12739P, the KCCM12739P strain and the aforementioned mutant strains in which random mutations were induced were cultured by the following method.

Each of the aforementioned strains was inoculated into a 96-Deep Well Plate-Dome (Bioneer) containing 400 µl of seed medium, and cultured in a plate shaking incubator (TAITEC) at 32°C and 1200 rpm for about 48 hours. The isoleucine concentrations of about 3000 cultured strains were respectively measured via a near-infrared (NIR) spectroscopic analyzer, and the top five mutant strains with improved isoleucine production ability compared to the parent strain KCCM12739P were selected.

### <Seed Medium (pH 7.0)>

Glucose 20 g, peptone 10 g, yeast extract 5 g, urea 1.5 g, KH₂PO₄ 4 g, K₂HPO₄ 8g, MgSO₄·7H₂O 0.5 g, biotin 100 µg, thiamine HCl 1000 µg, calcium pantothenate 2000 µg, nicotinamide 2000 µg (based on 1 L of distilled water)

To finally select strains with reproducibly increased L-isoleucine production ability from the five selected mutant strains, they were cultured and evaluated by the following method. The parent strain and the aforementioned mutant strains were inoculated into a 250 mL corner-baffled flask containing 25 mL of an isoleucine production medium, and then cultured with shaking at 32°C for 60 hours at 200 rpm. After the culture was completed, the L-isoleucine concentration in the culture broth was analyzed using HPLC, and the L-isoleucine production concentrations of each mutant strain are shown in Table 1 below.

### <Production Medium (pH 7.2)>

Glucose 10%, yeast extract 0.2%, ammonium sulfate 1.6%, monopotassium phosphate 0.1%, magnesium sulfate heptahydrate 0.1%, iron sulfate heptahydrate 10 mg/L, manganese sulfate monohydrate 10 mg/L, biotin 200 µg/L (based on 1 L of distilled water)

**[Table 1]**

| Strain name | L-isoleucine (g/L) |
|---|---|
| KCCM12739P | 2.0 |
| KCCM12739P_mt1 | 2.8 |
| KCCM12739P_mt2 | 2.6 |
| KCCM12739P_mt3 | 2.7 |
| **KCCM12739P_mt4** | **3.6** |
| KCCM12739P_mt5 | 2.5 |

Among the five selected mutant strains, KCCM12739P_mt4 was finally selected as the strain with significantly improved L-isoleucine production ability.

### EXAMPLE 2: Mutation identification using whole-genome sequencing (WGS)

Whole-genome sequencing (WGS) was performed for the KCCM12739P_mt4 strain selected in EXAMPLE 1-2 to analyze the sequence, and by comparing it with the parent strain KCCM12739P (SEQ ID NO: 52 to SEQ ID NO: 61), mutations that occurred in ten types of promoter regions were identified, and the sequences of the variant promoters containing the said mutations are as shown in Table 2 below.

**[Table 2]**

| SEQ ID NO: | Target Gene | Sequence (5'→3') |
|---|---|---|
| 1 | NCgl0867 | GACGTCAAAAGGTTTTAGCACTATAACGTTATGACGTTTTAGTG CTAAAGTGTGGCTTGTCAGATTCGTGTTGGTCGTGCGCCCGTA TGGTGATTTGAGACAGGGTTTAGGAGAATTAGTTCCATGTCGA ATCGCACGTCATCTTCACCGAAGAATTCAAAGCAGGAGCTGTG CAACTCGTCGTATCCTCAGGGCGCTCGGTCAAGGAAGTGGCC ACCGAGTTGGGTATCAAAGAAGGAACACTGAGCGCGTGGGTA AGAACCTGGAGGGCCGAGCATCCAGACGCCGGTGTGGATGAA CCAGGCCCAGTCGAATGGGCGAAATACAAGGCCCTGCAGGCT GAGAACGCCGCGTTGAAACGGGAAAACGAGTTTTGGGGAAAA GCCAGCGCCTTCT |
| 2 | NCgl1062 | CCCCCATTTGGTTCGCAGCCGATGAGGAGTTACGCACTATCGA CTCCATCGAAGGCCAAGCAAATTGGTCAATCAAGCGTGAATCC GGCTTCCATGAGCCAGTTGCCCGCCTCAAAGCTTGACCCATTT TCATAATCAGTGCCATGTGGGTTTACGGTTGATAC |
| 3 | NCgl2412 | CTAGGACACTGGAACCTAAATCCAGCGCGTCTGCCAATTCCGC CACAAGCGCTTAATGCGGGTTTTACTATACATGGTGTAGTCCC AAATTAACTAATCCGGGTGACCAACTGCACCGTAAAAAAAATTC GCTTATCGTAAATCAGCAGGTAGAATAACACTCT |
| 4 | NCgl1224 | ACAGCAATTAATCTGATTGCACCTGCTGCATAAATGTGGCTAG TCAAACACCGTCTAATTACATGTGTGTGGTAGAACAATAATGTA GTTGTCTGCCCAAGCGAGTTAAACTCCCACGATTTACAGTGGG GGGCAGACATCTTTTCACCAAAATTTTTACGAAAGGCGAGATTT TCTCCC |
| 5 | NCgl2581 | AAATTTGTGGGAACCGACCGACAAGTCCGCGGCCTGATCATG GACGTGCTGCGCAATGCCACCGCACCTGTGCCACTATCCGCG ATTGATGTCGTGTGGCCTGACGATGCCCAACGCTCCCGGGCG CTGTTTTCGCTCATTGAGGACGGACTCGCGGAACAAGATGAG GCGGGTTATTTCCACCTGCCACGATAAAACACTGCGCGCCTG CAAAAAACAGTAGGTTTAAGTA |
| 6 | NCgl2658 | AGCACAAATGCTTATTGCCGTAGATAACAATACGGAGCGGTTA CAAGTTGCGAAGCACCCTTTGCAAAGTTTTTAGCGGTACTTTA CAGCTCTTCACCCCCCATTAATGGGGTCTATGAAAGCGCGCAG CGTGACAAATCTGACCCCAGCAAAGCCCCCAATACTCCCCGA CTGGCCTTTATTAGTCTGCACTTACCACGTATCATGGAGGGTT GATAGCAGGGCACCATTAGCAGTCGCACCCCGATAGGAGTCG AATCTACAAGTGGAACCCCCGCTCACATACTCCACATTTTTTAG AACCCCTTTAAGGAATCGAACTTTAT |
| 7 | NCgl2046 | AGTACTTCGACGCAAAGACAAAACTTTTCTCCTGGCAATAAATA TGCGGATTTACTATGGAAACAAGATAGAAGATTGGATAGCGAA AGCTATCCTCAACTCGTGGAAAGTGTAGTGCCACAACCACAGT ATTGGCTAGAAAACAATCTATAGCATTGTTCTACAAAGAGCTTG TTGGAAATAAAACCTATGCCAAAGTAGGTGCAATTCTAGGAGT GTGGTATAATGGTCAACC |
| 8 | NCgl0780 | GGGGTGTGGTGGATACACATGGTTGTGTGTTCACCGCACCCT CTCTTTTTTTGTGTTTGTGGGGGTCTTGGGCCCCTTGTGCACG TGGCACCTGCGCCTGGGGGCAAGCGTCGACAAGCACAAACTT TTTGCTTAATTGAATCCTTTGCGCACCAATCAATGGGGGATCAA ATATAGTAGCTGC |
| 9 | NCgl1896 | CGCAAAGCTCACACCCACGAGCTAAAAATTCATATAGTTAAGA CAACATTTTTGGCTGTAAAAGACAGCCGTAAAAACCTCTTGCTC GTGTCAATTGTTCTTATCGGAATGTGGCTTGGGCGATTGTTAT GCAAAAGTTGTTAGGTTTTTTGCGTGGTTGTTGGGCCCCCAAA TGAGGGAAGAAGGTAACCTTGAACTCT |
| 10 | NCgl2356 | GTTTTCTAGATAACAAGGCCAGCACAGACCACCATATCTACGA CCCCAAAAACCGACTCCAAGCTCCGCGGCGACGAAGCCGCGC TCGCGCCACCGACCAAGCAGCCGGTCCAGGTTTAAAGATTTT GCTTTTCGACGCTCCCCTCCACCTCATTCAATGCGGCGGGAG GGGATTTCCTTGCATGTTAAGCCTATAGGAAAAAGTGTTTGCAT ATCACCCTTGTATTCGAACACGTGAGCGGGTAGAGTGGGTGG TAACAACACGGGGAAAGGGGGAAGACACC |

In the following examples, the effect of each variant promoter listed in Table 2 on the L-isoleucine production ability of a microorganism of the genus Corynebacterium was evaluated to identify effective factors affecting the L-isoleucine production ability.

### EXAMPLE 3: Construction of an L-isoleucine-producing strain introduced with a variant promoter

### Example 3-1: Construction of a recombinant vector for introducing a variant promoter

In order to insert each of the variant promoters of the NCgl0867, NCgl1062, NCgl2412, NCgl1224, NCgl2581, NCgl2658, NCgl2046, NCgl0780, NCgl1896, and NCgl2356 genes of Table 2 into KCCM12739P, a vector including the target mutation was constructed.

Specifically, the genomic DNA of the KCCM12739P_mt4 strain was extracted using a G-spin Total DNA Extraction Mini Kit (Intron, Cat. No 17045) according to the protocol provided in the kit, and PCR was performed using the genomic DNA as a template. The polymerase used was SolgTM Pfu-X DNA polymerase, and for the PCR conditions, after denaturation at 95°C for 4 minutes; after repeating 27 cycles of denaturation at 95°C for 30 seconds, annealing at 60°C for 30 seconds, and polymerization at 72°C for 50 seconds; a polymerization reaction was performed at 72°C for 5 minutes, and respective PCR products were obtained using SEQ ID NO: 12 and SEQ ID NO: 13; or SEQ ID NO: 14 and SEQ ID NO: 15; or SEQ ID NO: 16 and SEQ ID NO: 17; or SEQ ID NO: 18 and SEQ ID NO: 19; or SEQ ID NO: 20 and SEQ ID NO: 21; or SEQ ID NO: 22 and SEQ ID NO: 23; or SEQ ID NO: 24 and SEQ ID NO: 25; or SEQ ID NO: 26 and SEQ ID NO: 27; or SEQ ID NO: 28 and SEQ ID NO: 29; or SEQ ID NO: 30 and SEQ ID NO: 31. The primer sequences used in the above experiment are as shown in the following Table 3.

Recombinant plasmids were obtained by cloning the obtained mutation-introduced fragment and the pDC24 vector (SEQ ID NO: 11) treated with the restriction enzyme Smal using the Gibson assembly method (DG Gibson et al., NATURE METHODS, VOL.6 NO.5, MAY 2009, NEBuilder HiFi DNA Assembly Master Mix), and the vectors including each mutation-introduced fragment were named pDC24-Pn*_NCgl0867, pDC24-Pn*_NCgl1062, pDC24- Pn*_NCgl2412, pDC24- Pn*_NCgl1224, pDC24- Pn*_NCgl2581, pDC24- Pn*_NCgl2658, pDC24- Pn*_NCgl2046, pDC24-Pn*_NCgl0780, pDC24- Pn*_NCgl1896 and pDC24- Pn*_NCgl2356.

**[Table 3]**

| SEQ ID NO: | Name | Sequence (5'→3') |
|---|---|---|
| 11 | pDC24 | GTTCGCTTGCTGTCCATAAAACCGCCCAGTCTAGCTAT CGCCATGTAAGCCCACTGCAAGCTACCTGCTTTCTCTTT GCGCTTGCGTTTTCCCTTGTCCAGATAGCCCAGTAGCT GACATTCATCCGGGGTCAGCACCGTTTCTGCGGACTGG CTTTCTACGTGTTCCGCTTCCTTTAGCAGCCCTTGCGC CCTGAGTGCTTGCGGCAGCGTGAAGCTAGCTTTTATCG CCATTCGCCATTCAGGCTGCGCAACTGTTGGGAAGGG CGATCGGTGCGGGCCTCTTCGCTATTACGCCAGCTGG CGAAAGGGGGATGTGCTGCAAGGCGATTAAGTTGGGT AACGCCAGGGTTTTCCCAGTCACGACGTTGTAAAACGA |
| | | CGGCCAGTGAATTCGAGCTCGGTACCCGGGGATCCTC TAGAGTCGACCTGCAGGCATGCAAGCTTGGCGTAATCA TGGTCATAGCTGTTTCCTGTGTGAAATTGTTATCCGCTC ACAATTCCACACAACATACGAGCCGGAAGCATAAAGTG TAAAGCCTGGGGTGCCTAATGAGTGAGCTAACTCACAT TAATTGCGTTGCGCTCACTGCCCGCTTTCCAGTCGGGA AACCTGTCGTGCCAGCTGCATTAATGAATCGGCCAACG CGCGGGGAGAGGCGGTTTGCGTATTGGGCGCTCTTCC GCTTCCTCGCTCACTGACTCGCTGCGCTCGGTCGTTCG GCTGCGGCGAGCGGTATCAGCTCACTCAAAGGCGGTA ATACGGTTATCCACAGAATCAGGGGATAACGCAGGAAA GAACATGTGAGCAAAAGGCCAGCAAAAGGCCAGGAAC CGTAAAAAGGCCGCGTTGCTGGCGTTTTTCCATAGGCT CCGCCCCCCTGACGAGCATCACAAAAATCGACGCTCAA GTCAGAGGTGGCGAAACCCGACAGGACTATAAAGATAC CAGGCGTTTCCCCCTGGAAGCTCCCTCGTGCGCTCTCC TGTTCCGACCCTGCCGCTTACCGGATACCTGTCCGCCT TTCTCCCTTCGGGAAGCGTGGCGCTTTCTCAATGCTCA CGCTGTAGGTATCTCAGTTCGGTGTAGGTCGTTCGCTC CAAGCTGGGCTGTGTGCACGAACCCCCCGTTCAGCCC GACCGCTGCGCCTTATCCGGTAACTATCGTCTTGAGTC CAACCCGGTAAGACACGACTTATCGCCACTGGCAGCAG CCACTGGTAACAGGATTAGCAGAGCGAGGTATGTAGGC GGTGCTACAGAGTTCTTGAAGTGGTGGCCTAACTACGG CTACACTAGAAGGACAGTATTTGGTATCTGCGCTCTGC TGAAGCCAGTTACCTTCGGAAAAAGAGTTGGTAGCTCT TGATCCGGCAAACAAACCACCGCTGGTAGCGGTGGTTT TTTTGTTTGCAAGCAGCAGATTACGCGCAGAAAAAAAG GATCTCAAGAAGATCCTTTGATCTTTTCTACGGGGTCTG ACGCTCAGTGGAACGAAAACTCACGTTAAGGGATTTTG GTCATGAGATTATCAAAAAGGATCTTCACCTAGATCCTT TTGGGGTGGGCGAAGAACTCCAGCATGAGATCCCCGC GCTGGAGGATCATCCAGCCCTGATAGAAACAGAAGCCA CTGGAGCACCTCAAAAACACCATCATACACTAAATCAGT AAGTTGGCAGCATCACCCGACGCACTTTGCGCCGAATA AATACCTGTGACGGAAGATCACTTCGCAGAATAAATAAA |
| | | TCCTGGTGTCCCTGTTGATACCGGGAAGCCCTGGGCC AACTTTTGGCGAAAATGAGACGTTGATCGGCACGTAAG AGGTTCCAACTTTCACCATAATGAAATAAGATCACTACC GGGCGTATTTTTTGAGTTATCGAGATTTTCAGGAGCTGA TAGAAACAGAAGCCACTGGAGCACCTCAAAAACACCAT CATACACTAAATCAGTAAGTTGGCAGCATCACCCGACG CACTTTGCGCCGAATAAATACCTGTGACGGAAGATCAC TTCGCAGAATAAATAAATCCTGGTGTCCCTGTTGATACC GGGAAGCCCTGGGCCAACTTTTGGCGAAAATGAGACG TTGATCGGCACGTAAGAGGTTCCAACTTTCACCATAATG AAATAAGATCACTACCGGGCGTATTTTTTGAGTTATCGA GATTTTCAGGAGCTCTTTGGCATCGTCTCTCGCCTGTC CCCTCAGTTCAGTAATTTCCTGCATTTGCCTGTTTCCAG TCGGTAGATATTCCACAAAACAGCAGGGAAGCAGCGCT TTTCCGCTGCATAACCCTGCTTCGGGGTCATTATAGCG ATTTTTTCGGTATATCCATCCTTTTTCGCACGATATACAG GATTTTGCCAAAGGGTTCGTGTAGACTTTCCTTGGTGTA TCCAACGGCGTCAGCCGGGCAGGATAGGTGAAGTAGG CCCACCCGCGAGCGGGTGTTCCTTCTTCACTGTCCCTT ATTCGCACCTGGCGGTGCTCAACGGGAATCCTGCTCTG CGAGGCTGGCCGGCTACCGCCGGCGTAACAGATGAGG GCAAGCGGATGGCTGATGAAACCAAGCCAACCAGGAA GGGCAGCCCACCTATCAAGGTGTACTGCCTTCCAGACG AACGAAGAGCGATTGAGGAAAAGGCGGCGGCGGCCG GCATGAGCCTGTCGGCCTACCTGCTGGCCGTCGGCCA GGGCTACAAAATCACGGGCGTCGTGGACTATGAGCAC GTCCGCGAGGGCGTCCCGGAAAACGATTCCGAAGCCC AACCTTTCATAGAAGGCGGCGGTGGAATCGAAATCTCG TGATGGCAGGTTGGGCGTCGCTTGGTCGGTCATTTCGA AAAAGGTTAGGAATACGGTTAGCCATTTGCCTGCTTTTA TATAGTTCATATGGGATTCACCTTTATGTTGATAAGAAAT AAAAGAAAATGCCAATAGGATATCGGCATTTTCTTTTGC GTTTTTATTTGTTAACTGTTAATTGTCCTTGTTCAAGGAT GCTGTCTTTGACAACAGATGTTTTCTTGCCTTTGATGTT CAGCAGGAAGCTCGGCGCAAACGTTGATTGTTTGTCTG CGTAGAATCCTCTGTTTGTCATATAGCTTGTAATCACGA |
| | | CATTGTTTCCTTTCGCTTGAGGTACAGCGAAGTGTGAG TAAGTAAAGGTTACATCGTTAGGCGGATCAAGATCCATT TTTAACACAAGGCCAGTTTTGTTCAGCGGCTTGTATGG GCCAGTTAAAGAATTAGAAACATAACCAAGCATGTAAAT ATCGTTAGACGTAATGCCGTCAATCGTCATTTTTGATCC GCGGGAGTCAGTGAACAGGTACCATTTGCCGTTCATTT TAAAGACGTTCGCGCGTTCAATTTCATCTGTTACTGTGT TAGATGCAATCAGCGGTTTCATCACTTTTTTCAGTGTGT AATCATCGTTTAGCTCAATCATACCGAGAGCGCCGTTT GCTAACTCAGCCGTGCGTTTTTTATCGCTTTGCAGAAGT TTTTGACTTTCTTGACGGAAGAATGATGTGCTTTTGCCA TAGTATGCTTTGTTAAATAAAGATTCTTCGCCTTGGTAG CCATCTTCAGTTCCAGTGTTTGCTTCAAATACTAAGTAT TTGTGGCCTTTATCTTCTACGTAGTGAGGATCTCTCAGC GTATGGTTGTCGCCTGAGCTGTAGTTGCCTTCATCGAT GAACTGCTGTACATTTTGATACGTTTTTCCGTCACCGTC AAAGATTGATTTATAATCCTCTACACCGTTGATGTTCAA AGAGCTGTCTGATGCTGATACGTTAACTTGTGCAGTTGT CAGTGTTTGTTTGCCGTAATGTTTACCGGAGAAATCAGT GTAGAATAAACGGATTTTTCCGTCAGATGTAAATGTGGC TGAACCTGACCATTCTTGTGTTTGGTCTTTTAGGATAGA ATCATTTGCATCGAATTTGTCGCTGTCTTTAAAGACGCG GCCAGCGTTTTTCCAGCTGTCAATAGAAGTTTCGCCGA CTTTTTGATAGAACATGTAAATCGATGTGTCATCCGCAT TTTTAGGATCTCCGGCTAATGCAAAGACGATGTGGTAG CCGTGATAGTTTGCGACAGTGCCGTCAGCGTTTTGTAA TGGCCAGCTGTCCCAAACGTCCAGGCCTTTTGCAGAAG AGATATTTTTAATTGTGGACGAATCAAATTCAGAAACTT GATATTTTTCATTTTTTTGCTGTTCAGGGATTTGCAGCAT ATCATGGCGTGTAATATGGGAAATGCCGTATGTTTCCTT ATATGGCTTTTGGTTCGTTTCTTTCGCAAACGCTTGAGT TGCGCCTCCTGCCAGCAGTGCGGTAGTAAAGGTTAATA CTGTTGCTTGTTTTGCAAACTTTTTGATGTTCATCGTTCA TGTCTCCTTTTTTATGTACTGTGTTAGCGGTCTGCTTCT TCCAGCCCTCCTGTTTGAAGATGGCAAGTTAGTTACGC ACAATAAAAAAAGACCTAAAATATGTAAGGGGTGACGC |
| | | CAAAGTATACACTTTGCCCTTTACACATTTTAGGTCTTG CCTGCTTTATCAGTAACAAACCCGCGCGATTTACTTTTC GACCTCATTCTATTAGACTCTCGTTTGGATTGCAACTGG TCTATTTTCCTCTTTTGTTTGATAGAAAATCATAAAAGGA TTTGCAGACTACGGGCCTAAAGAACTAAAAAATCTATCT GTTTCTTTTCATTCTCTGTATTTTTTATAGTTTCTGTTGC ATGGGCATAAAGTTGCCTTTTTAATCACAATTCAGAAAA TATCATAATATCTCATTTCACTAAATAATAGTGAACGGCA GGTATATGTGATGGGTTAAAAAGGATCACCCCAGAGTC CCGCTCAGAAGAACTCGTCAAGAAGGCGATAGAAGGC GATGCGCTGCGAATCGGGAGCGGCGATACCGTAAAGC ACGAGGAAGCGGTCAGCCCATTCGCCGCCAAGCTCTT CAGCAATATCACGGGTAGCCAACGCTATGTCCTGATAG CGGTCCGCCACACCCAGCCGGCCACAGTCGATGAATC CAGAAAAGCGGCCATTTTCCACCATGATATTCGGCAAG CAGGCATCGCCATGGGTCACGACGAGATCCTCGCCGT CGGGCATCCGCGCCTTGAGCCTGGCGAACAGTTCGGC TGGCGCGAGCCCCTGATGCTCTTCGTCCAGATCATCCT GATCGACAAGACCGGCTTCCATCCGAGTACGTGCTCGC TCGATGCGATGTTTCGCTTGGTGGTCGAATGGGCAGGT AGCCGGATCAAGCGTATGCAGCCGCCGCATTGCATCA GCCATGATGGATACTTTCTCGGCAGGAGCAAGGTGAGA TGACAGGAGATCCTGCCCCGGCACTTCGCCCAATAGCA GCCAGTCCCTTCCCGCTTCAGTGACAACGTCGAGACAG CTGCGCAAGGAACGCCCGTCGTGGCCAGCCACGATAG CCGCGCTGCCTCGTCTTGGAGTTCATTCAGGGCACCG GACAGGTCGGTCTTGACAAAAAGAACCGGGCGCCCCT GCGCTGACAGCCGGAACACGGCGGCATCAGAGCAGCC GATTGTCTGTTGTGCCCAGTCATAGCCGAATAGCCTCT CCACCCAAGCGGCCGGAGAACCTGCGTGCAATCCATC TTGTTCAATCATGCGAAACGATCCTCATCCTGTCTCTTG ATCAGATCTTGATCCCCTGCGCCATCAGATCCTTGGCG GCAAGAAAGCCATCCAGTTTACTTTGCAGGGCTTCCCA ACCTTACCAGAGGGCGCCCCAGCTGGCAATTCCG |
| 12 | Pn*_NCgl0867_F | tgaattcgagctcggtacccATCGACAAACGCTTCAACAA |
| 13 | Pn*_NCgl0867_R | gtcgactctagaggatccccCCCGTTTCAACGCGGCGTTC |
| 14 | Pn*_NCgl1062_F | tgaattcgagctcggtacccCATTTCGGCGCCCATCACCA |
| 15 | Pn*_NCgl1062_R | gtcgactctagaggatccccAGGTGGAAGGCCGCCTGAGT |
| 16 | Pn*_NCgl2412_F | tgaattcgagctcggtacccATTCTCCACCGGCTCCCACC |
| 17 | Pn*_NCgl2412_R | gtcgactctagaggatccccGAGCTGAACGAAGCTATCTT |
| 18 | Pn*_NCgl1224_F | tgaattcgagctcggtacccATCCCCGTGCTCAAAGTCGT |
| 19 | Pn*_NCgl1224_R | gtcgactctagaggatccccCAGCGGTGGTCTTGACCTCG |
| 20 | Pn*_NCgl2581_F | tgaattcgagctcggtacccGTTGGGTGCTCTTATCTGCA |
| 21 | Pn*_NCgl2581_R | gtcgactctagaggatccccGATACCAATGAACACCGGAT |
| 22 | Pn*_NCgl2658_F | tgaattcgagctcggtacccATGCGTCTCAGCCACTGGGA |
| 23 | Pn*_NCgl2658_R | gtcgactctagaggatccccAGATTCCTGCTGGACCTGCA |
| 24 | Pn*_NCgl2046_F | tgaattcgagctcggtacccTTCATCGTCATCATCGCTGGT |
| 25 | Pn*_NCgl2046_R | gtcgactctagaggatccccGTAGGAACGAACATCCTGCA |
| 26 | Pn*_NCgl0780_F | tgaattcgagctcggtacccCTGTTTCGAGATGAG |
| 27 | Pn*_NCgl0780_R | gtcgactctagaggatccccCCCGCCAACGCAATA |
| 28 | Pn*_NCgl1896_F | tgaattcgagctcggtacccCTGACCGACCAGGAA |
| 29 | Pn*_NCgl1896_R | gtcgactctagaggatccccGGATGAGTCTCGGTT |
| 30 | Pn*_NCgl2356_F | tgaattcgagctcggtacccCCACTGCCATGGCTG |
| 31 | Pn*_NCgl2356_R | gtcgactctagaggatccccGTTGAGGCGGGCGAG |

### EXAMPLE 3-2: Construction of an L-isoleucine-producing strain introduced with a variant promoter

The 10 types of vectors prepared in the above Example 3-1 were transformed into the isoleucine-producing strain KCCM12739P by an electroporation method (Appl. Microbiol. Biotechnol. (1999) 52:541-545), and strains in which the vector was inserted onto a chromosome by recombination of a homologous sequence were selected using a kanamycin medium. Thereafter, strains into which a variant promoter was introduced were confirmed from among the transformants in which a second recombination was completed, through PCR using primers of SEQ ID NO: 32 and SEQ ID NO: 33; SEQ ID NO: 34 and SEQ ID NO: 35; or SEQ ID NO: 36 and SEQ ID NO: 37; or SEQ ID NO: 38 and SEQ ID NO: 39; or SEQ ID NO: 40 and SEQ ID NO: 41; or SEQ ID NO: 42 and SEQ ID NO: 43; or SEQ ID NO: 44 and SEQ ID NO: 45; or SEQ ID NO: 46 and SEQ ID NO: 47; or SEQ ID NO: 48 and SEQ ID NO: 49; or SEQ ID NO: 50 and SEQ ID NO: 51. The PCR was performed in the same manner as in Example 3-1. The recombinant strains were designated as KCCM12739PΔPn::Pn*_NCgl0867, KCCM12739PΔPn::Pn*_NCgl1062, KCCM12739PΔPn::Pn*_NCgl2412, KCCM12739PΔPn::Pn*_NCgl224, KCCM12739PΔPn::Pn*_NCgl2581, KCCM12739PΔPn::Pn*_NCgl2658, KCCM12739PΔPn::Pn*_NCgl2046, KCCM12739PΔPn::Pn*_NCgl0780, KCCM12739PΔPn::Pn*_NCgl1896, and KCCM12739PΔPn::Pn*_NCgl2356, respectively. The primer pairs used for the confirmation are as shown in Table 4 below.

**[Table 4]**

| SEQ ID NO | Name | Sequence (5'->3') |
|---|---|---|
| 32 | pNCgl0867_F | AATCAATCTTCGCACTCG |
| 33 | pNCgl0867_R | TCTCTTGCTCGATGAACG |
| 34 | pNCgl1062_F | ACTCGAATTCACCGAAAC |
| 35 | pNCgl1062_R | CTCATCCACGTCTACTGT |
| 36 | pNCgl2412_F | CTACGGACACATGGAATA |
| 37 | pNCgl2412_R | TCTTCCACATCCATCGTC |
| 38 | pNCgl1224_F | TAAAGACCGAAACACTCG |
| 39 | pNCgl1224_R | CGTTGGTGAAGATTTCTG |
| 40 | pNCgl2581_F | CTTATCGACGTCTCCCTT |
| 41 | pNCgl2581_R | TGGGAGCGAGTTCTTGGA |
| 42 | pNCgl2658_F | ATTGATGACACTTCAGG |
| 43 | pNCgl2658_R | CAACACCATAACGGATCA |
| 44 | pNCgl2046_F | ACGCAGAGTCTGCTTGAAC |
| 45 | pNCgl2046_R | ACAGGAACATAGATGCG |
| 46 | pNCgl0780_F | GTCCCTAGTACGAGAGGACC |
| 47 | pNCgl0780_R | TTATGCGGCGAATTAACGAT |
| 48 | pNCgl1896_F | CAAGTCCCGTTTCTTTGATC |
| 49 | pNCgl1896_R | CCGCCATTTTTCCTTCTCTC |
| 50 | pNCgl2356_F | TAGCTCGACTTGGCGCTGAA |
| 51 | pNCgl2356_R | GTTTTTATTGGCGGTGGTGA |

### EXAMPLE 4. Evaluation of L-isoleucine production ability of the L-isoleucine-producing strain introduced with a variant promoter

To evaluate the L-isoleucine production ability of the strains prepared in the above Example 3-2 at a flask level, the strains were cultured and evaluated by the following method. After a parent strain and the mutant strains were inoculated into a 250 mL corner-baffled flask containing 25 mL of an isoleucine production medium, they were shake-cultured at 200 rpm at 32°C for 60 hours.

After completion of the culture, the L-isoleucine production amount was measured using high-performance liquid chromatography (HPLC), and the analysis results are shown in Table 5 below.

### <Production Medium (pH 7.2)>

Glucose 10%, Yeast extract 0.2%, Ammonium sulfate 1.6%, Potassium phosphate monobasic 0.1%, Magnesium sulfate heptahydrate 0.1%, Iron sulfate heptahydrate 10 mg/L, Manganese sulfate monohydrate 10 mg/L, Biotin 200 µg/L (based on 1 L of distilled water)

**[Table 5]**

| Comparison of isoleucine production ability of L-isoleucine-producing strains introduced with a variant promoter (flask titer evaluation, 60 hours) | |
|---|---|
| Strain Name | L-isoleucine concentration (g/L) |
| KCCM12739P | 2.0 |
| KCCM12739PΔPn::Pn*_NCgl0867 | 2.2 |
| KCCM12739PΔPn::Pn*_NCgl1062 | 2.1 |
| **KCCM12739PΔPn::Pn*_NCgl2412** | **2.9** |
| KCCM12739PΔPn::Pn*_NCgl1224 | 2.0 |
| KCCM12739PΔPn::Pn*_NCgl2581 | 2.0 |
| KCCM12739PΔPn::Pn*_NCgl2658 | 2.0 |
| **KCCM12739PΔPn::Pn*_NCgl2046** | **3.4** |
| KCCM12739PΔPn::Pn*_NCgl0780 | 2.3 |
| **KCCM12739PΔPn::Pn*_NCgl1896** | **2.6** |
| KCCM12739PΔPn::Pn*_NCgl2356 | 2.2 |

As shown in Table 5 above, the isoleucine production ability of the strain into which a variant promoter was introduced was increased or showed an equivalent level compared to the parent strain. In particular, it was confirmed that the isoleucine production abilities of 'KCCM12739PΔPn::Pn*_NCgl2412', 'KCCM12739PΔPn::Pn*_NCgl2046', and 'KCCM12739PΔPn::Pn*_NCgl1896' were significantly improved compared to the parent strain KCCM12739P.

Through this, it could be confirmed that L-isoleucine could be produced more efficiently by introducing a variant promoter that regulates the expression of the NCgl2412 gene, a variant promoter that regulates the expression of the NCgl2046 gene, and/or a variant promoter that regulates the expression of the NCgl1896 gene.

From the above description, those skilled in the art will understand that the present application can be implemented in other specific forms without altering its technical concept or essential characteristics. In this regard, it should be understood that the embodiments described above are illustrative in all respects and not restrictive. The scope of this application should be interpreted to include all changes or modifications derived from the meaning and scope of the following claims and their equivalents, rather than the detailed description above.

## Claims

1. A polynucleotide, comprising
a nucleotide sequence wherein the nucleotide at position 99 is substituted with cytosine (C) in the nucleotide sequence of SEQ ID NO: 54;
a nucleotide sequence wherein the nucleotide at position 217 is substituted with thymine (T), the nucleotide at position 218 is substituted with guanine (G), the nucleotide at position 219 is substituted with thymine (T), the nucleotide at position 220 is substituted with guanine (G), the nucleotide at position 221 is substituted with guanine (G), the nucleotide at position 224 is substituted with thymine (T), the nucleotide at position 226 is substituted with adenine (A), and the nucleotide at position 228 is substituted with guanine (G) in the nucleotide sequence of SEQ ID NO: 58; or
a nucleotide sequence wherein the nucleotide at position 155 is substituted with thymine (T), the nucleotide at position 163 is substituted with guanine (G), the nucleotide at position 164 is substituted with guanine (G), and the nucleotide at position 165 is substituted with guanine (G) in the nucleotide sequence of SEQ ID NO: 60.

2. The polynucleotide according to claim 1, wherein the polynucleotide has a promoter activity.

3. The polynucleotide according to claim 1, wherein the polynucleotide has 80% or more and less than 100% sequence identity to the nucleotide sequences of SEQ ID NO: 54, SEQ ID NO: 58, or SEQ ID NO: 60, respectively.

4. The polynucleotide according to claim 1, wherein the polynucleotide comprises a nucleotide sequence of SEQ ID NO: 3, a nucleotide sequence of SEQ ID NO: 7, or a nucleotide sequence of SEQ ID NO: 9.

5. An expression cassette comprising the polynucleotide according to claim 1 and a target gene.

6. A microorganism of the genus *Corynebacterium,* comprising
the polynucleotide according to any one of claims 1 to 4; or
the polynucleotide and a target gene operably linked thereto.

7. The microorganism according to claim 6, wherein the microorganism of the genus *Corynebacterium* is *Corynebacterium glutamicum.*

8. A method for producing L-isoleucine, comprising culturing the microorganism according to claim 6 in a medium.

9. The method for producing L-isoleucine according to claim 8, further comprising recovering L-isoleucine from the cultured medium or the microorganism.

10. Use of the microorganism according to claim 6 or 7 for producing L-isoleucine.
